# EUROPEAN PATENT APPLICATION

(11) **EP 1 610 253 A1**
(43) Date of publication of application: **28.12.2005**
(21) Application number: 04023262.1
(22) Date of filing: 30.09.2004
(51) Int. Cl.: G06F 19/00

(54) **Method for acquisition of data in structured format**

(30) Priority: 17.05.2004 IT PI20040034
(71) Applicant: Medexpert S.r.l., 56122 Pisa (IT)
(72) Inventor: Cannavo, Giovanni, 56122 Pisa (IT); Mastroroberto, Luigi, 40100 Bologna (IT); Velucchi, Mario, 56121 Pisa (IT)
(74) Representative: Celestino, Marco

(57) **Abstract**

Method for acquisition and transmission of data, in particular of a medical examination, comprising one or more interface files for acquisition of data comprising a predetermined number of fields, where the data concerning a medical examiner's report comprise the personal data of a doctor who draws up the report and of the damaged person as well as other data relative to the medical examination, a file in structured format that contains said data and is suited to be transmitted file in said structured format to an insurance company. The content of the fields is extracted automatically from the interface file, acquired in the system, treated according to inner decoding tables in order to transform it in said file in structured format, then sent to the companies automatically, according to a customized, shared and agreed upon protocol.

## Description

### Field of the invention.

The present invention relates to a method for acquisition of data in structured format, in particular for medical examinations and for subsequent data transfer to authorised bodies.

### Description of the prior art.

The need is felt of an evaluation method by use in medical examinations concerning both automobile liability insurances (RCA) and accident insurances (Accident insurance)

As known in the field of damages to the public in automobile liability insurances, most of activity relates to disputes on "small damages", "small disablements" or on the so called "micropermanent" impairments. In particular, about 80% of medical examinations for insurance companies relate to cases where permanent biological damages are recognized within the value of 3%.

Medical examiners are trying to give a real significance to such values wondering whether or not they relate to actual pathological phenomena that cause the health of the damaged persons to a permanent worsening. Only in the affirmative, in fact, the damages should be recognised as if the injuries were concretely and indisputably disabling in a permanent way.

Often, the recognition of such permanent damages depend on a personal evaluation of the medical examiner, sometimes affected by an insufficient, or absent, or aspecific clinical picture, and that is not necessarily derivable from the accident consequences. This type of approach can give cause for speculations, which can affect seriously the amount of the damages.

Normally when "micropermanent" injuries are observed, the usual instruments (examination of the medical documentation provided by the damaged persons person, subjectivity of the damaged persons person's statements and clinical objectivity) are insufficient for distinguishing those cases where a legitimate recognition can be made, in which a minimum indemnifiable impairment can be acknowledged, from those that instead are to be considered as speculations.

The presently adopted approach is also affected by an unhomogeneity of the data exchanged between the medical examiner that works on behalf of an insurance company and the insurance company same and for the absence of unified criteria in the exchange of such data.

Such data, furthermore, must be put in a national database, so called "accidents register", and, without a method that assists this step, relevant input and data treatment costs arise.

Therefore, it is necessary to reorganize the approach to the medical examinations, partly improving the methodology of analysis, partly providing instruments that are more precise with respect to those now available.

The above problems are in particular felt for micropermanent impairments, which are largely the most common cases treated.

Of course, and even more so, the same methodology is used for all medical examinations of both public liability insurance and accident insurances, for all the levels up to 100% of disablement.

### Summary of the invention

It is a feature of the present invention to provide a method for acquisition and transmission of data for medical examiner's reports that is much easier and quicker with respect to existing methods.

Another object is to provide a method that allows a medical examiner to detect in a rational and quick way the data relative to the medical examiner's report and to send them electronically to another medical examiner that draws up the report for the insurance company.

A further object of the method for acquisition of data in structured format, as well as for allowing the determination of damages, is to supply an accident database, for example a central accident records office.

Another feature of the present invention is to allow a decisional staff to manage the accident, in order to determine concretely all the elements useful for a correct consideration of the single elements making up permanent damages.

Another feature of the present invention is to integrate different professional competences (such as those of a fiduciary physician and of an expert) so that an assessor, once collected all the data and opinions, can settle at best the case.

A further feature of the present invention is to improve the exchange of data between medical examiner and assessor, concerning the privacy of the damaged persons, with particular reference to "sensible data" when the lawyers of the damaged persons require to the insurance company a copy of the report written by its fiduciary medical examiner.

These and other features are accomplished with one exemplary method for acquisition and transmission of data, in particular of a medical examination, comprising the steps of:
- providing one or more interface files for acquisition of data comprising a predetermined number of fields;
- typing in the fields of said or each interface file data concerning the medical examiner's report comprising the personal data of a doctor that draws up the medical examiner's report and of the damaged person, and the other data relative to the medical examiner's report;
- producing a file in structured format that contains said starting data from said or each interface file;
- transmitting said file in structured format to the insurance company.

In particular, the content of the fields is extracted automatically from the interface file, acquired in the system, treated according to inner decoding tables in order to transforming it in said file in structured format, then sent to the companies automatically, according to a customized, shared and agreed upon protocol.

Preferably said interface files use a univocal medical examiner's report model, capable of respecting the privacy, characterised in that it comprises:
- a first part containing the medical report concerning clinical, anamnestic and documental data, so called "sensible data", collected by the fiduciary medical expert during its verification, said first part being copyble to the damaged person under request;
- a second part comprising an objective evaluation representing only the opinion of the examiner on the case, which is kept secret by the insurance company.

In particular, said first part of the medical examiner's report comprises personal data of the medical examiner that carries out the medical examiner's report, the personal data of the damaged person, comprised the profession thereof for possible cross verifications, the remote anamnesis, the analysis of the accident, the clinical history and the examination of the documentation, the symptomatology, the objective examination; said second part of the medical examiner's report, which remains secret, can comprise the personal data of the medical examiner and the epicrisis report, that comprises the personal opinions of the examiner, the diagnosis, the damaged person's request, the judgment and the comments to it.

Advantageously the interface files consists of a medical examiner's report model comprising:
- a formatted text document (for example Word®) which works as start and selection menu, between the choice of drawing up a medical examiner's report in RCA or in accident insurance;
- a formatted text document (for example Word®) that works as RCA model of the medical examiner's report;
- a formatted text document (for example Word®) that works as accident insurance model of the medical examiner's report;
- a electronic sheet (for example Excel®), which gathers the list of all the Italyn Hospitals, each marked with a code, and that works as reference for drawing up said formatted text documents;
wherein said or each formatted text document comprises:
- free text input fields;
- forced text input fields as pull down menu, where entries can be put in input according to a predetermined coding;
- paragraph portions modifiable by the examiner, both about content and format, where a doctor can write what he/she considers useful or can remove them or leave them empty;
- not modifiable fixed parts, for example the titles of the sections.

Advantageously said forced text input fields allow to save the medical examiner's report in a format selected from the group:
- a usuale printable/displayable format;
- a text line, where the digited data concerning the fields are separated by a symbol.

In particular several lines of text (records) are merged through a "merge" function in a single text table, then imported for further uses, in a database table, for example an Access® table.

Alternatively, said or each document of formatted text, once compiled, can be encoded into another format in order to be sent easily through data communication means.

Alternatively, the medical examination can be executed in a version to be drawn up "on-line" via web interface, allowing to send the data directly.

Advantageously the medical examiner's report and its coding is not a static but a dynamic product with possibility of periodical updates in the arrangement of the paragraphs, record entries, codings, additions, deletions, variations, in order to adapt to current needs.

### Brief description of the drawings.

The invention will be made clearer with the following description of an exemplary embodiment thereof, exemplifying but not limitative, with reference to the attached drawings wherein:
- figure 1 shows a diagrammatical view of a block diagram that describes the method;
- figure 2 shows the character string to insert in the document of formatted text;
- figure 3 shows the specification of the content of the fields and relative admitted value;
- figures 4 and 5 shows the content of the menu as pull down menu for forced text input fields;
- figure 6 shows the printout of the file of formatted text concerning the medical examiner RCA report;
- figure 7 shows the printout of the file of formatted text concerning the medical examiner accident insurance report.

### Description of an preferred exemplary embodiments.

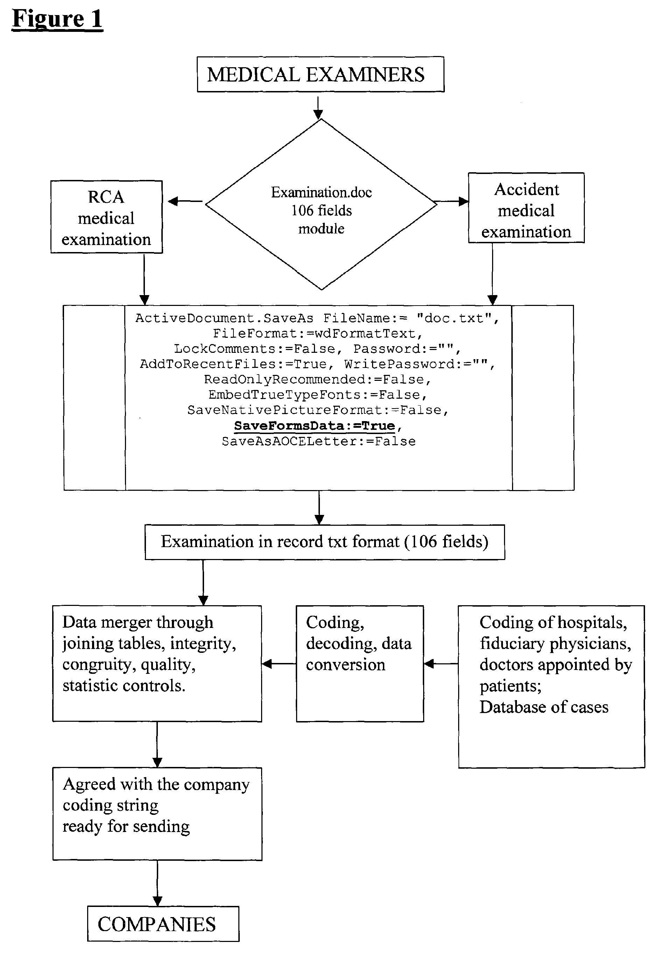

Figure 1 shows a block diagram of the method for acquisition of data in structured format.

Such diagram starts from when a doctor begins drawing up the medical examiner's report, filling in the fields of either a file of formatted text (for example Word®) working as as RCA model of the report or a file of formatted text document (for example Word®) that works as accident insurance model of the report.

After having inserted the data relative to the medical examiner's report the file of formatted text is saved, thus starting the execution of a VisualBasic code, contained in the file same.

The result of running this code is a 106 fields "record txt" format file.

A this point the data inserted can be compared and completed by data contained in different databases such as data about Hospitals, fiduciary medical experts, witness medical experts, or database available within the company.

At the same time the data are merged through merging tables and then their reliability, congruity, quality is checked.

The method for acquisition provides a coded output string previously agreed with the company and ready to be sent to the company and to be put in a national database called "central accident records office".

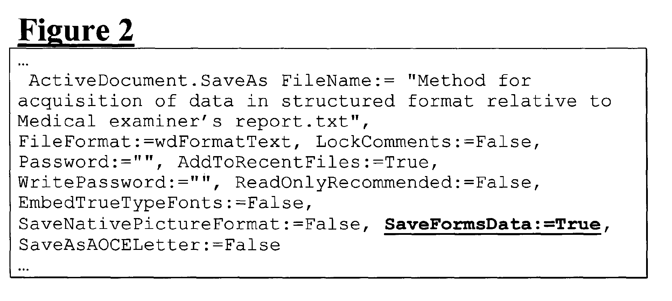

In figure 2 the code in Visual Basic is shown as inserted in the formatted text document for example in Word® format, which follows the sequence described in figure 1.

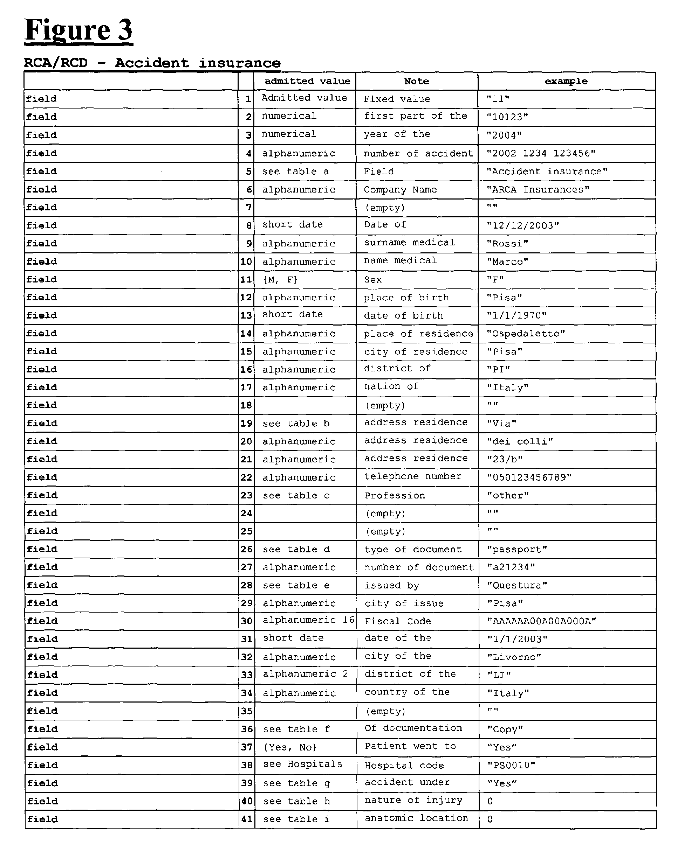

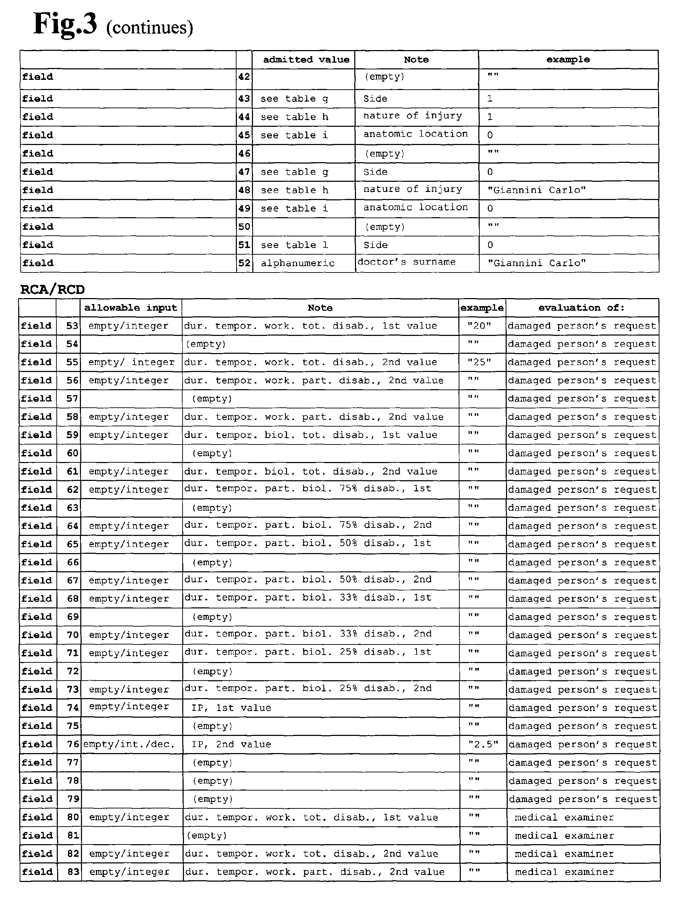

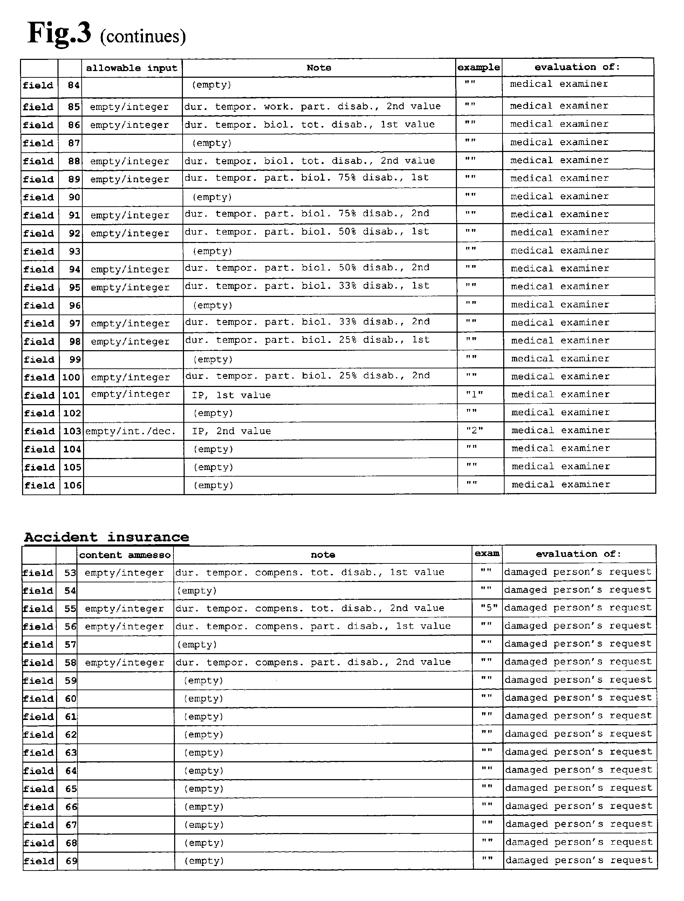

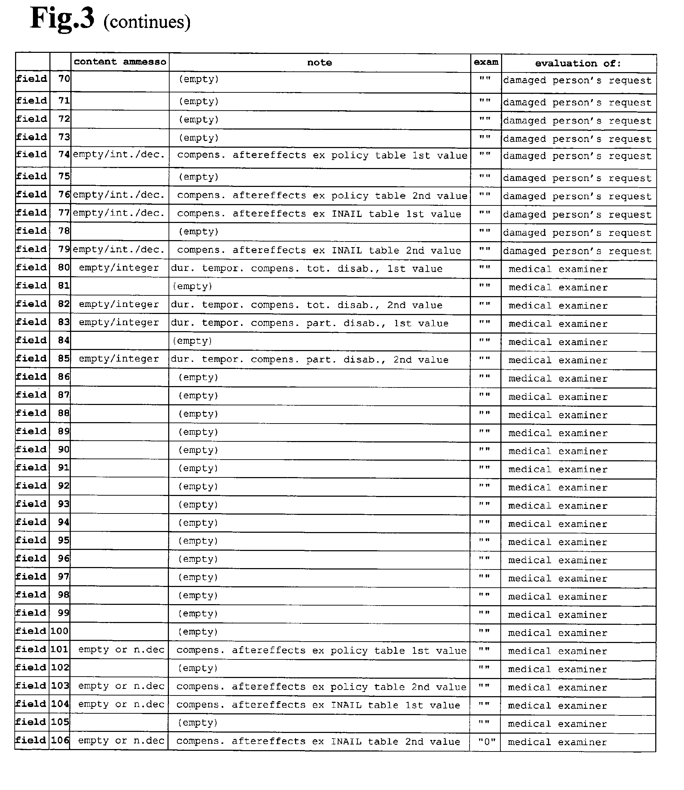

A example of medical examiner's report in txt record format, for example of 106 fields, is described in figure 3. It represents the specification of the content of the fields and the relative admitted value. From field 1 to field 52 included, the content of the fields coincides for the medical examination of both RCA and accident insurance reports; from line 53 to 106 they are clearly separated from each other.

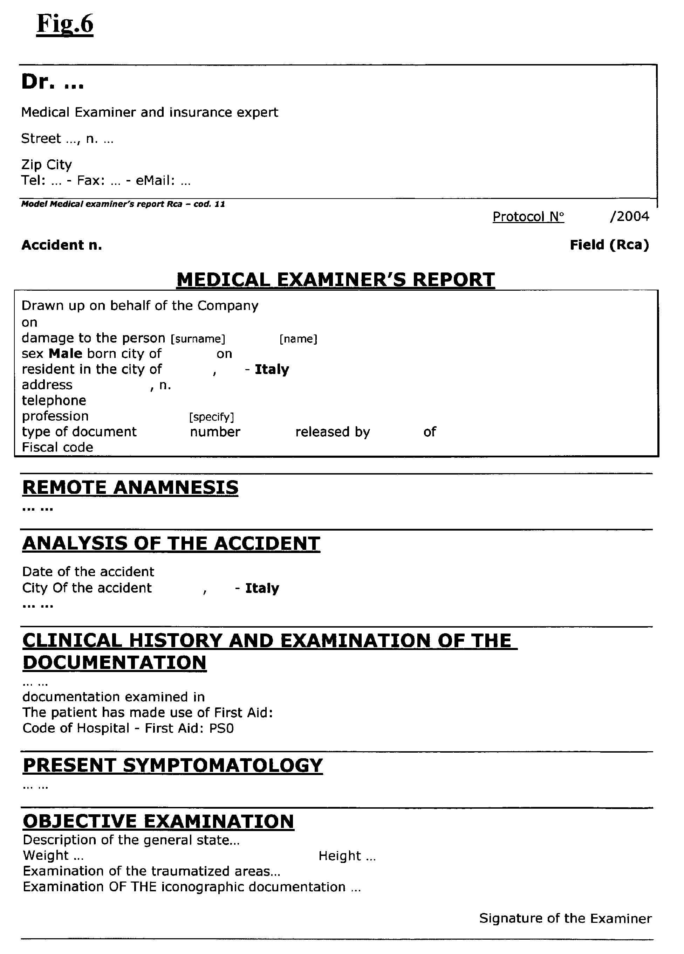

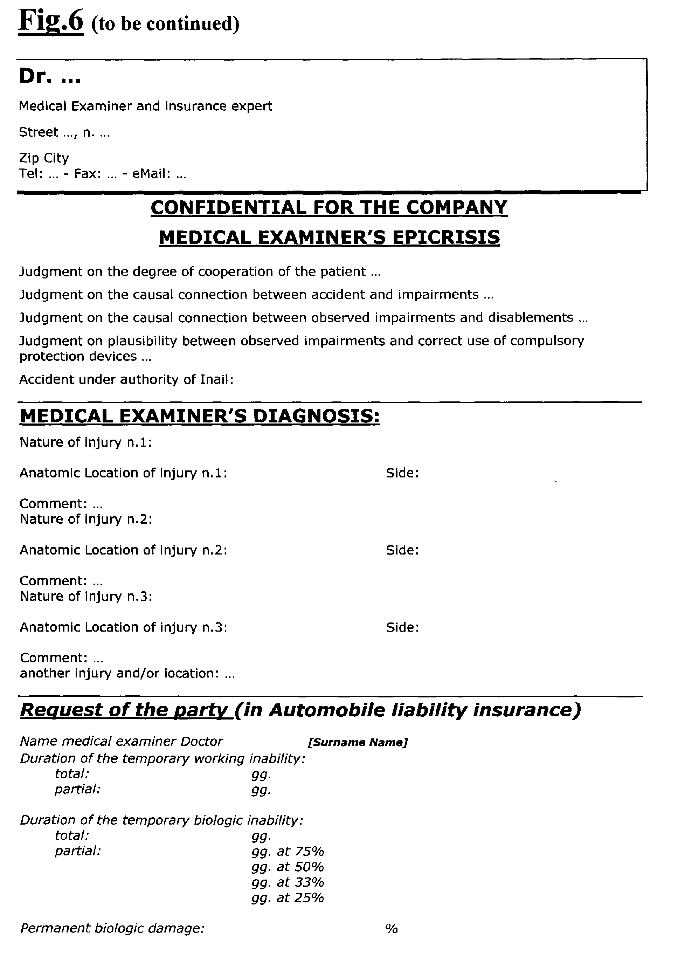

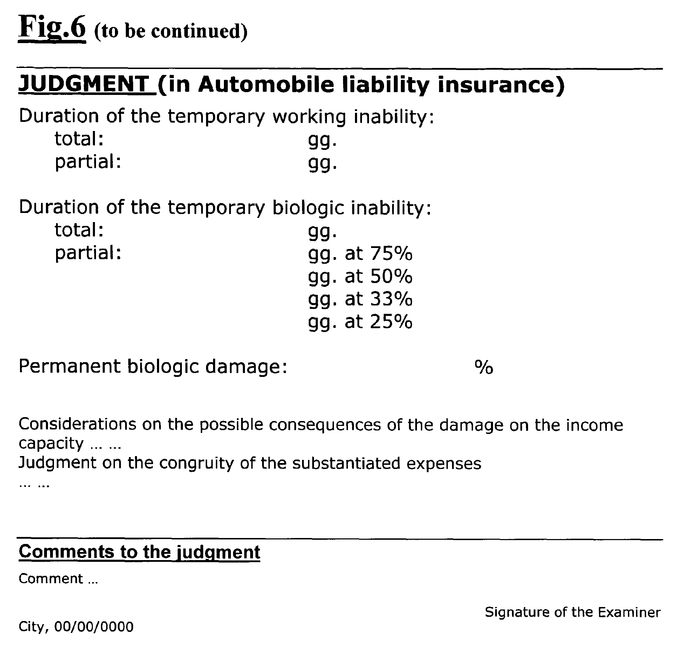

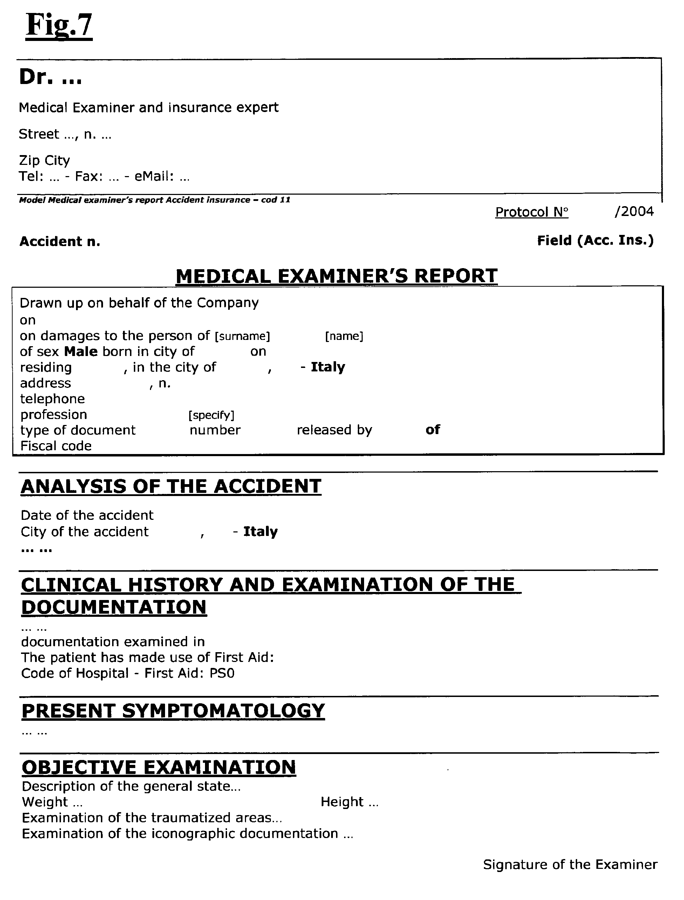

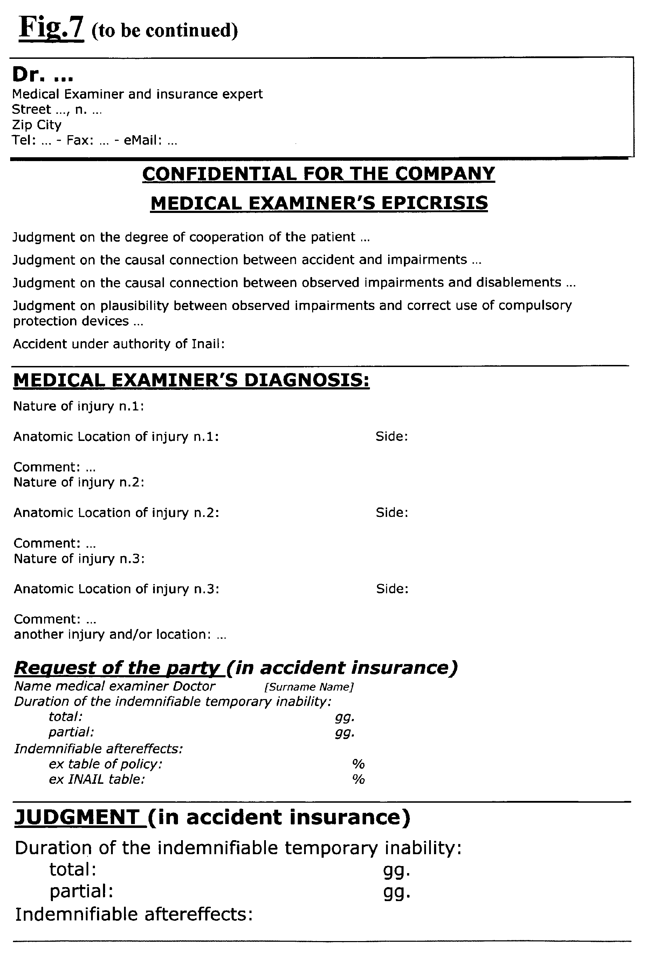

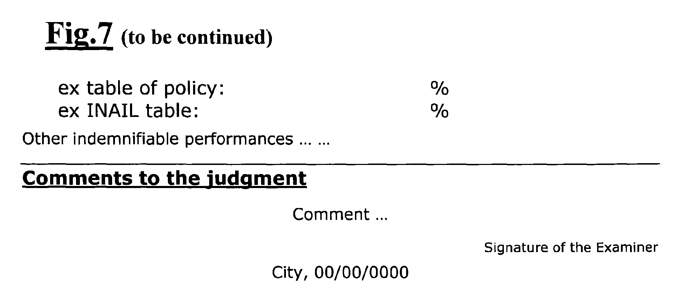

In figures 6 and 7 the printouts of the files are shown that refer respectively to the medical examiner's RCA report and to the medical examiner's accident insurance report. They contain free text input fields, forced text input fields arranged as pull down menu where entries can be put in according to a predetermined coding, parts of paragraph modifiable by the examiner, concerning both the content and the format, where a doctor can write what he/she considers useful or can remove them or leave them empty, as well as not modifiable fixed parts, for example the titles of the sections.

In particular in the heading the medical examiner's report shows the medical examiner's personal datathat carries out the medical examiner's report. Then the fields are shown of the medical examiner's report and then the RCA.

In the next section, the medical examiner's report allows to put in the personal data of the damaged person, where in addition to the place and date of birth, the profession of the parents in case of childs, of the residence and the domicile of the damaged person, a detailed information of the kind of job of the damaged person is put in, as well as the administrative features of the job, in particular whether he/she is a self-employed worker or an employee, requiring in the latter case the name of the body or the company where he/she is employed. Such data allow assessing the period of absence of the damaged person from the workplace.

Further section of the medical examiner's report is the "remote anamnesis", both clinical and traumatological, which is often critical for a correct interpretation of the medical examiner on the consequences of a determined accident. For compilation of this field, in case of doubts, the medical examiner can consult an existing database called "Central accident records office" for an analysis on possible previous accidents. This database is continually updated and completed with the aid of the insurance companies that must provide as soon as they have a new record on a determined accident with injuries, and allows the company to determine which other company had dealt with the previous accident and also the numerical reference of the previous accident same.

Then the section follows of "Data, time and modes of the accident", which should be essential for reaching a correct interpretation of the case, determining any elements of a possible contestation of the requests of the damaged person and checking the reliability thereof, analysing the causal connection. Immediately after, a field follows that requires information on whether the accident has been declared as occurred on the workplace.

The following section relates to "Clinical history and examination of the documentation" that consists of the analysis of the documentation and of the clinical history referred by the damaged person, which allows testing if and up to which point the information given by previous medical reports are compatible with a starting condition, with a chronological relation and with a continuity of the phenomena.

Then, the sections follow of "Present Symptomatology" and "Objective Examination" that relate to the summary of the direct verification on damaged person, completed with a direct verification of possible verification through images on which the requests of the damaged person are based.

At this point the first part of the medical examiner's report ends, which related specifically to "sensible data" of the examined patient, followed by the signature of a doctor below, so that, if requested by the damaged person or by an attorney thereof, if the request is allowable, this part of the medical examiner's report can be given.

The second part of the medical examiner's report relates to the "Medical Examiner's Epicrisis", which is a confidential part and cannot be given to the damaged person or the attorney thereof, and is the conclusion of the verification, where the medical examiner provides to the assessor its convincements on the case. In this step the medical examiner-fiduciary expert must explain to the assessor if the case is legitimate or if some points can be questioned. This part of the medical examiner's report starts with the "judgment on the degree of cooperation of the patient during the medical examination". Then, a "judgment on the causal connection between accident and injuries" follows, which is the Judgment on a doctor in the evaluation if the accident examined was capable of causing impairments and also if such impairments are really compatible with the traumatogenetic events.

Immediately after, a field follows concerning a "judgment on the causal connection between impairments and disablements" for establishing an admissible correlation between an observed injury and an overall disabling status presumably deriving from it.

The medical examiner's report continues with a "judgment of compatibility between assessed impairments and correct use of compulsory protection devices". The acquisition of this field is of substantial importance, since, according to the Case Law, if the damaged person had to use such compulsory protection devices, but did not use them, then it can be assumed that the damages are higher than those caused if the damaged person had used such compulsory protection devices, thus reducing the amount of the compensation.

Then, a section is provided in the model comprising the medical examiner's report diagnosis, concerning any disablements which are object of the evaluation. In this case the model can be filled in both through coded fields with pull down menus, choosing the nature of disablement, the location of the disablement in the body, and the injured side of the body, and also through free fields, where additional data not comprised in the pull down menu can be put in. The medical examiner's report model provides as a default three different impairments subsequent to a same accident, even if a further free field, to be filled in by writing, if more than three impairments were present.
the menu are arranged to allow extraction of all the data necessary for completing the ISVAP and INAIL databases, but also for carrying out statistic and more detailed controls.

A "judgment and notes" section follows which sums up all what was considered in the previous steps of the examination.

In this section there is a "temporary inability" that represents the consequences that the medical examiner must determine, according to actual manifestations developed from the injury, in particular a "temporary biologic inability", total or partial, which quantifies for how long and how much an injured subject was prevented from carrying out the common operations of its daily life, and the "temporary working inability", total or partial, which quantifies for how long and how much the subject has suffered impairments on the capacity to attend to the usual business, which would have produced utility and income.

The medical examiner's report continues with a "evaluation of the permanent biologic damage", drawn up in accordance to the analysis of the causal connection, of the compatibility of the injuries, etc., and comprises also a field "estimate of the INAIL biological damage" for a thorough and more prudent decision on how to manage the case.

Then, in the medical examiner's report a field is present called "opinion on the congruity of the substantiated or declared expenses", to be filled in after analysis on the patient or after question to the patient for establishing which expenses for medical treatments have been born and of which type. This way, if, an "expenses list" is delivered to the insurance company only after the medical examination, the assessor may compare that expenses list with the opinion of the fiduciary medical examiner.

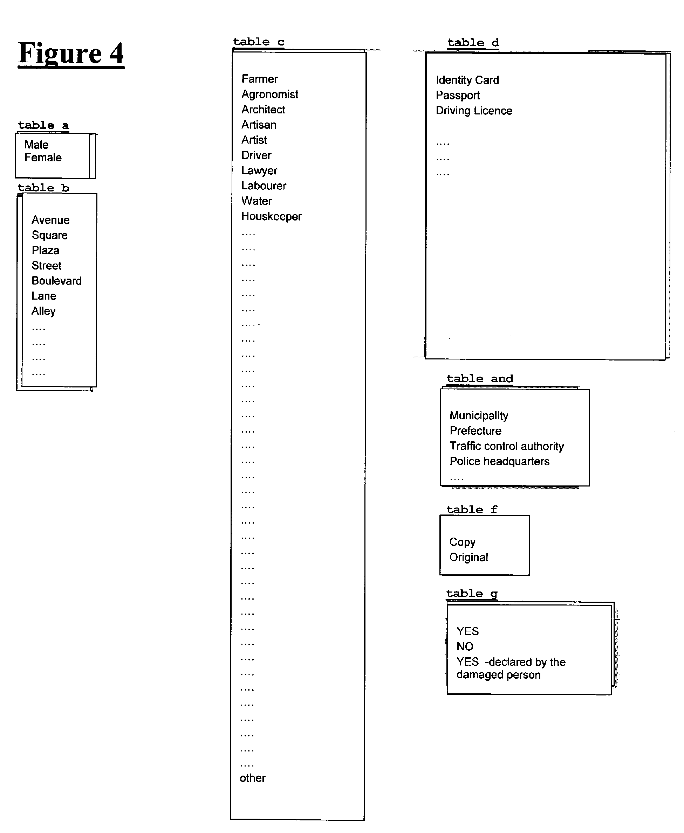

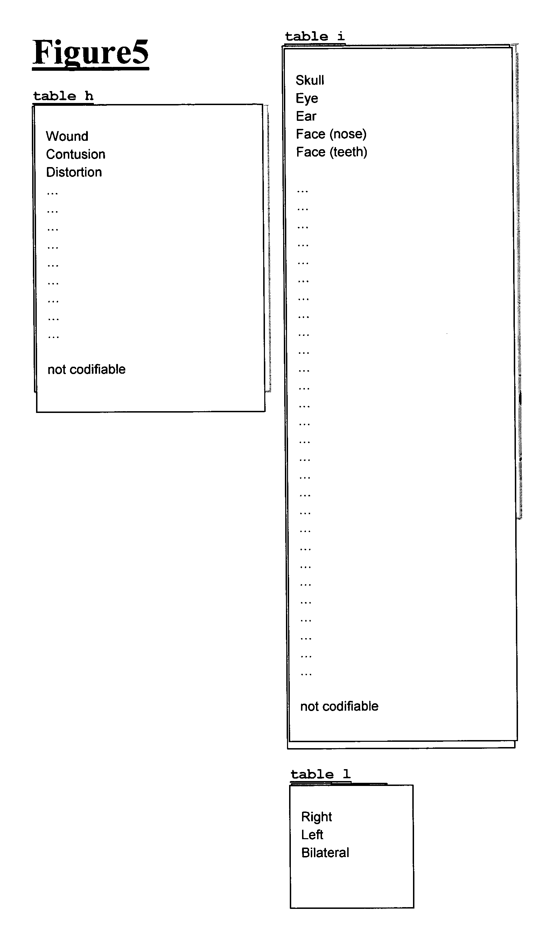

Figures 4 and 5 show the entries that can be chosen in forced text input fields through a respective pull down menu in the medical examinations described in figures 6 and 7.

The foregoing description of a specific embodiment will so fully reveal the invention according to the conceptual point of view, so that others, by applying current knowledge, will be able to modify and/or adapt for various applications such an embodiment without further research and without parting from the invention, and it is therefore to be understood that such adaptations and modifications will have to be considered as equivalent to the specific embodiment. The means and the materials to realise the different functions described herein could have a different nature without, for this reason, departing from the field of the invention. It is to be understood that the phraseology or terminology employed herein is for the purpose of description and not of limitation.

## Claims

1. Method for acquisition and transmission of data, in particular of a medical examination, according to the present invention comprising the steps of:
- providing one or more interface files for acquisition of data comprising a predetermined number of fields;
- typing in the fields of said or each interface file the data concerning the medical examiner's report comprising the personal data of a doctor that draws up the medical examiner's report and of the damaged person, and the other data relative to the medical examiner's report;
- producing a file in structured format that contains said starting data from said or each interface file;
- transmitting said file in structured format to an insurance company.

2. Method for acquisition and transmission of data, according to claim 1, wherein the content of the fields is extracted automatically from the interface file, acquired in the system, treated according to inner decoding tables in order to transforming it in said file in structured format, then sent to the companies automatically, according to a customized, shared and agreed upon protocol.

3. Method for acquisition and transmission of data, according to claim 1, wherein said interface files use a univocal medical examiner's report model, capable of respecting the privacy, **characterised in that** it comprises:
- a first part containing the medical report concerning clinical, anamnestic and documental data, so called "sensible data" collected by the fiduciary medical expert during its verification, said first part being copyble to the damaged person under request;
- a second part comprising an objective evaluation representing only the opinion of the examiner on the case, which is kept secret by the insurance company.

4. Method for acquisition and transmission of data, according to claim 3, wherein said first part of the medical examiner's report comprises the medical examiner's personal datathat carries out the medical examiner's report, the personal data of the damaged person, comprised the profession for possible cross verifications, the remote anamnesis, the analysis of the accident, the clinical history and the examination of the documentation, the present symptomatology, the objective examination; said second part of the medical examiner's report, which remains secret, can comprise the medical examiner's personal dataand the medical examiner's epicrisis that comprises the personal opinions of the examiner, the medical examiner's diagnosis, the damaged person's request, the judgment and the comments to the judgment.

5. Method for acquisition and transmission of data, according to claim 1, wherein the interface files consists of a medical examiner's report model that comprises:
- a formatted text document (for example Word®) which works as start and selection menu, between the choice of drawing up a RCA or in accident insurance medical examiner's report;
- a formatted text document (for example Word®) that works as RCA model of the medical examiner's report;
- a formatted text document (for example Word®) that works as accident insurance model of the medical examiner's report;
- a electronic sheet (for example Excel®), which gathers the list of all the Italyn Hospitals, each marked with a code, and that works as reference for drawing up said formatted text documents.

6. Method for acquisition and transmission of data, according to claim 5, wherein said or each formatted text document comprises:
- free text input fields;
- forced text input fields as pull down menu, where entries can be put in according to a predetermined coding;
- paragraph portions modifiable by the examiner, concerning both the content and the format, where a doctor can write what he/she considers useful or can remove them or leave them empty;
- not modifiable fixed parts, for example the titles of the sections.

7. Method for acquisition and transmission of data, according to claim 6, wherein said forced text input fields allow to save the medical examiner's report in a format selected from the group:
- a printable/displayable format;
- a text line, where the digited data concerning the fields are separated by a symbol.

8. Method for acquisition and transmission of data, according to claim 7, wherein several lines of text (records) are merged through a "merge" function in a single text table, then imported for further uses, in a database table, for example through Access®.

9. Method for acquisition and transmission of data, according to claim 1, wherein said or each interface file, once compiled, can be encoded into another format in order to be sent easily through data communication means.

10. Method for acquisition and transmission of data, according to claim 1, wherein the medical examination can be executed in a version to be drawn up "on-line" via web interface, allowing to send the data directly.

11. Method for acquisition and transmission of data, according to claim 1, wherein the medical examiner's report and its coding is not a static but a dynamic product with possibility of periodical updates in the arrangement of the paragraphs, record entries, codings, additions, deletions, variations, in order to adapt to current needs.
